## Europäisches Patentamt
## European Patent Office
## Office européen des brevets

(11) Publication number: **0 039 967**
**B1**

(12) **EUROPEAN PATENT SPECIFICATION**

(45) Date of publication of patent specification: **17.10.84**

(21) Application number: **81200470.3**

(22) Date of filing: **04.05.81**

(51) Int. Cl.³: **A 61 K 31/43, A 61 K 9/00**

(54) Solvate of amoxicillin, process for the preparation thereof and process for the preparation of injectable preparations from this solvate.

(30) Priority: **08.05.80 NL 8002636**

(43) Date of publication of application:
**18.11.81 Bulletin 81/46**

(45) Publication of the grant of the patent:
**17.10.84 Bulletin 84/42**

(84) Designated Contracting States:
**AT BE CH DE FR GB IT LI LU NL SE**

(56) References cited:
**EP-A-0 009 845**
**EP-A-0 012 495**
**GB-A-1 465 694**
**GB-A-1 538 903**
**GB-A-2 000 970**

**Kirk-Othmer, Vol.2, p. 879**
**Ullmanns, Vol. 16, p. 304 and Vol. 19, p. 641**

(73) Proprietor: **Gist - Brocades N.V.**
**Wateringseweg 1 P.O. Box 1**
**NL-2600 MA Delft (NL)**

(72) Inventor: **Heitman, Herwarth**
**Von Boisotring 31**
**NL-2722 AA Zoetermeer (NL)**
Inventor: **Van der Drift, Johannes Karel**
**Marterstraat 39**
**NL-2623 ED Delft (NL)**
Inventor: **Leenderts, Everardus Johannes Anthonius M.**
**Jasmijn 16**
**NL-3161 CM Rhoon (NL)**
Inventor: **Grootveld, Herman Hendrik**
**Prinses Beatrixstraat 27**
**NL-2731 GA Benthuizen (NL)**

(74) Representative: **Schmieman, Johannes Hendrik et al**
**Gist-Brocades N.V. Patent Department P.O. Box 1**
**NL-2600 MA Delft (NL)**

Courier Press, Leamington Spa, England.

## Description

This invention relates to a crystalline solvate of sodium amoxicillin, a process for its preparation and to the preparation of therapeutically useful injectable preparations from this solvate.

The literature, more particularly the patent literature of recent years, shows that great efforts have been made to find amoxicillin preparations which in dry form are sufficiently stable and which provide a suitable starting material for stable injectable solutions. The prior literature also shows that the chemical and physical properties of amoxicillin differ from those of semi-synthetic penicillins known hitherto to such a degree that the methods commonly used for the preparation of injectable solutions of those penicillins cannot be applied generally for the formation of injectable preparations of amoxicillin.

Suggested solutions include modified preparations from the usual alkali metal salts of amoxicillin and on the other hand the use of salts with different cations.

A method to obtain an amoxicillin preparation by freeze-drying which can be constituted into an injectable preparation, is disclosed in British patent No. 1527557. According to this patent sodium amoxicillin is freeze-dried in an aqueous solvent system to which a secondary or tertiary alkanol with four or five carbon atoms has been added as a stabiliser.

British patent No. 1466553 discloses a process for the preparation of the choline salt and the N-methyl-D-glucamine salt of amoxicillin, whereas in British patent No. 1464065 the preparation of the arginine salt of amoxicillin is described. Said salts of amoxicillin would result in new non-toxic amoxicillin preparations for parenteral administration to humans with preservation of the antibiotic activities. It is also stated in the latter patent that neither amoxicillin as such nor the salts known hitherto can be administered satisfactorily by the parenteral route. The Japanese published application 51032723 (Kokai) discloses the preparation of a suitable injectable solution comprising amoxicillin and the sodium salt of glycine.

British patent No. 1471235 discloses a process for the preparation of salts of D-$\alpha$-carboxyamino-p-hydroxybenzyl penicillin, intended for the preparation of satisfactory injectable amoxicillin compositions. It is also stated here explicitly that the preparation of injectable preparations of amoxicillin had been found to be much more difficult than initially anticipated, due to the decomposition of amoxicillins salts in aqueous solutions.

According to said patent mixtures of the sodium salt of amoxicillin and the disodium salt of D-$\alpha$-carboxyaminop-hydroxybenzyl penicillin should preferably be applied.

Canadian patent No. 1045549 describes a process for the production of a stable preparation of amoxicillin from which stable injectable preparations can be obtained which are well tolerated on administration. This preparation consists of a powder that is easily constituted into an injectable preparation by addition of an aqueous vehicle. The powder consists of minute particles of amoxicillin trihydrate coated with a dispersion agent with the ratio of amoxicillin trihydrate to dispersion agent being from 1000:1 to 20:1. The minute particles should have a mean diameter of 2 $\mu$m to 20 $\mu$m and at least 95% of them should have a diameter between 0.5 $\mu$m and 50 $\mu$m, whereas 10 to 100% of the surface should be coated with the dispersion agent.

For similar reasons research has been directed to amoxicillin derivatives which yield amoxicillin on decomposition in the body. This principle is illustrated by the disclosures of U.S. patents nos. 4035381 and 4029805.

The preparation of solvates of alkali metal or alkaline earth metal salts of amoxicillin with amides is described in British patent No. 1465694. Preferred amides are those of the formula $RCONR_1R_2$ (in which R represents a hydrogen atom or a methyl group and $R_1$ and $R_2$ each represents a hydrogen atom or a methyl or ethyl group) and urea. The Examples show that only dimethylformamide and dimethylacetamide have been actually applied. In the preparation of the solvates amoxicillin trihydrate serves as a starting material which is dehydrated first by treatment with an alkanol. This requires a considerable time of stirring and results in a gelatinous product that is difficult to handle. According to three of the four Examples a diethylamine salt is formed first and this is subsequently converted into the sodium salt. The solvates formed with amides are not always well defined products with a constant composition. The use of N-methyl-2-pyrrolidone in compositions for topical application having a penicillin as the active ingredient, is described in GB—A—1,538,903.

Surprisingly it has been found that the sodium salt of amoxicillin forms a crystalline solvate with N-methyl-2-pyrrolidone which crystalline solvate is a well defined product with a constant composition. It can be obtained in a simple manner in a stable crystalline form which is suited to be constituted into a useful injectable preparation. The invention provides as a new substance the solvate of sodium amoxicillin with N-methyl-2-pyrrolidone.

According to a feature of the present invention the new solvate is prepared by reacting amoxicillin, preferably the trihydrate, with a solution containing a source of sodium ions and subsequently causing the solvate to crystallise in the presence of N-methyl-2-pyrrolidone by addition of one or more organic solvents.

The reaction between amoxicillin and the source of sodium ions is preferably carried out in a polar organic solvent. The reaction may for example suitably be carried out in N-methyl-2-pyrrolidone or a mixture thereof with a polar organic solvent. A suitable solvent mixture is for instance a mixture of N-methyl-2-pyrrolidone with an alcohol having at most 6 carbon atoms, for example ethanol.

The reaction and the crystallisation are preferably carried out at a temperature between —10° and 40°C, more particularly between 0 and 25°C. Suitable sources of sodium ions are sodium compounds already known in respect of the preparation of other semi synthetic penicillins. Sodium 2-ethylhexanoate is preferably used. Other examples of sources of sodium ions are sodium ethoxide, sodium hydroxide, sodium carbonate and sodium bicarbonate. If desired, the sodium ethylhexanoate can be prepared in situ from the base and the acid.

Various organic solvents can be used to effect crystallisation of the solvate. Solvents already known in the preparation of penicillin salts are generally suitable for the purpose. Preferred solvents are halogenated hydrocarbons such as dichloromethane and 1,2-dichloroethane, esters such as ethyl acetate and methyl acetate, alcohols such as ethanol and propan-2-ol, and nitriles such as acetonitrile.

According to a most preferred feature of the invention an amount of ethanol is added first and then propan-2-ol.

The following Examples illustrate the preparation of solvates according to the invention.

### Example I

48.5 ml (304 mmole) of 2-ethylhexanoic acid were added to 150 ml of N-methyl-2-pyrrolidone. To the resulting solution 11.1 g (278 mmole) of sodium hydroxide were added. The mixture thus obtained was stirred for at least 8 hours under anhydrous conditions after which 150 ml of N-methyl-2-pyrrolidone were added.

To the solution thus prepared 100 g (238 mmole) of amoxicillin trihydrate were added at 10°C. After being stirred for one hour the solution was filtered. Some seeding crystals were added to the solution thus obtained and then 335 ml of dichloromethane were added dropwise at 20°C in the course of 1.5 hours. The precipitate obtained was isolated by filtration and repeatedly washed with mixtures of N-methyl-2-pyrrolidone and dichloromethane with an increasing content of dichloromethane and finally with dichloromethane. The product was dired in vacuo at 30°C to a constant weight. The yield was 95 g (81.9%). The product contained 73.1% of amoxicillin (calculated as the free acid), 20% of N-methyl-2-pyrrolidone, 0.7% of dichloromethane, 1.2% of decomposition products and 0.9% of water.

### Example II

The procedure of Example I was followed with substitution of the dichloromethane by 400 ml of ethyl acetate. The yield was 98 g (84.5%). The product contained 72.2% of amoxicillin (calculated as the free acid), 21% of N-methyl-2-pyrrolidone, 0.5% of ethyl acetate, 0.5% of decomposition products and 0.7% of water.

### Example III

The procedure of Example I was followed with substitution of the dichloromethane by 300 ml of ethanol. The yield was 44 g (38%). The product contained 73.7% of amoxicillin (calculated as the free acid), 20% of N-methyl-2-pyrrolidone, 2% of ethanol, 0.5% of decomposition products and 0.4% of water. The non-crystallised amoxicillin could be recovered in a simple way as the trihydrate.

### Example IV

The procedure of Example I was followed with substitution of the dichloromethane by 400 ml of acetonitrile. The yield was 91 g (78.4%). The product contained 73.1% of amoxicillin (calculated as the free acid), 20% of N-methyl-2-pyrrolidone, 1% of acetonitrile, 0.8% of decomposition products and 0.3% of water.

### Example V

The procedure of Example I was followed with substitution of dichloromethane by 450 ml of propan-2-ol. The yield was 63 g (54.3%). The product contained 70.3% of amoxicillin (calculated as the free acid).

### Example VI

11.1 g (278 mmole) of sodium hydroxide were added to a mixture of 100 ml of absolute ethanol and 48.5 ml (304 mmole) of 2-ethylhexanoic acid. The mixture obtained was refluxed for one hour and subsequently cooled to 10°C. To the solution thus prepared 200 ml of N-methyl-2-pyrrolidone and 70 g (166.9 mol) of amoxicillin trihydrate were added. After stirring for about 45 minutes at a temperature of 10 to 20°C the clear solution was filtered. The filter was washed with 50 ml of N-methyl-2-pyrrolidone. To the solution thus obtained some seeding crystals were added. In the course of 1.5 hours 150 ml of absolute ethanol were added dropwise at 20°C and then over the same time 400 ml of propan-2-ol were added, after which the solution was stirred for two hours at about 20°C. The precipitate obtained was isolated by filtration and washed with 100 ml of a mixture (1/1) of N-methyl-2-pyrrolidone and absolute ethanol and then with absolute ethanol. The product was dried in vacuo at 30°C to a constant weight. The yield was 63 g (77.6%). The product contained 74.2% of amoxicillin (calculated as the free acid), 19% of N-methyl-2-pyrrolidone, 2% of ethanol, 0.3% of propan-2-ol and 0.4% of water.

The amoxicillin content was determined by the ferri hydroxamate method. The percentage of decomposition products was determined by mercurimetric titration.

The percentages of N-methyl-2-pyrrolidone and other solvents were determined by means of NMR spectroscopy and gas chromatography.

The water content was determined by the method of Karl Fischer.

The analysis results show that in the product obtained the molecular ratio amoxicillin:N-methyl-2-pyrrolidone always is approximately 1:1. X-ray diffraction of the crystals confirms that the substance contains only one type of crystals. The X-ray photographs of the substances obtained according to the various Examples were nearly identical.

The following Table shows, by way of example, the results obtained with the substance prepared according to Example III.

| d | I | d | I |
|---|---|---|---|
| 1.00 | 2 | 0.391 | 1 |
| 0.94 | 1 | 0.379 | 1B |
| 0.90 | 8 | 0.377 | 1 |
| 0.76 | 4 | 0.370 | 4 |
| 0.64 | 1 | 0.359 | 1 |
| 0.62 | 1 | 0.355 | 2 |
| 0.575 | 10B | 0.344 | 1 |
| 0.56 | 6 | 0.333 | 2 |
| 0.54 | 4 | 0.317 | 1B |
| 0.51 | 6 | 0.311 | 3 |
| 0.50 | 2B | 0.304 | 1 |
| 0.46 | 1 | 0.300 | 1 |
| 0.44 | 2 | 0.293 | 3 |
| 0.425 | 1B | 0.288 | 1 |
| 0.400 | 1 | 0.213 | 1 |

d = distance between lattice planes in nanometers

I = intensity (range 1—10)

B = line is broader than normal

Lines with an intensity less than 0.07 are omitted.

From the above data it can be calculated that the substance is monoclinic with the following unit cell dimensions.

$$a = 2.01077 \text{ nm}$$

$$b = 1.01129 \text{ nm}$$

$$c = 1.16451 \text{ nm}$$

$$\beta = 98.60°$$

A number of preparations were stored from one to three weeks in tightly closed bottles at 65°C. Significant changes in amoxicillin content and the percentage of decomposition products did not occur.

The invention also includes the preparation of injectable compositions from the solvate of sodium amoxicillin and N-methyl-2-pyrrolidone by a method customarily employed in pharmacy. The injectable composition may, for instance, be prepared by adding distilled, sterile, pyrogen-free water and other optional auxiliary substances to the dry solvate and sterilizing the resulting solution by the usual method. Alternatively, a sterile solvate is prepared by sterile filtration and the said solvate is made into an injectable solution under aseptic conditions.

Examples of suitable amoxicillin concentrations in the injectable liquid are 200 and 50 mg/ml, respectively for intramuscular and intravenous administration. Suitable dosages of amoxicillin for parenteral administration in adult humans range from 750 to 4500 mg daily.

**Claims for the Contracting States: BE, CH, DE, FR, GB, IT, Li, LU, NL, SE**

1. Crystalline solvate of sodium amoxicillin with N-methyl-2-pyrrolidone.

2. Process for the preparation of the solvate claimed in claim 1, characterized in that amoxicillin is reacted with a solution of a source of sodium ions and the solvate is subsequently caused to crystallize by addition of one or more organic solvents in the presence of N-methyl-2-pyrrolidone.

3. Process according to claim 2, characterized in that amoxicillin is used in the trihydrate form.

4. Process according to claim 2 or 3, characterized in that the reaction between amoxicillin and the source of sodium ions is carried out in a polar organic solvent.

5. Process according to claim 4, characterized in that N-methyl-2-pyrrolidone or a mixture thereof with a polar organic solvent is used as the polar organic solvent.

6. Process according to claim 5, characterized in that a mixture of N-methyl-2-pyrrolidone with an alcohol having at most 6 carbon atoms is used as the polar organic solvent.

7. Process according to any one of claims 2 to 6, characterized in that the reaction and the crystallization of the solvate are carried out at a temperature between —10 and +40° centigrade.

8. Process according to claim 7, characterized in that the reaction and the crystallization of the solvate are carried out at a temperature between 0 and 25° centigrade.

9. Process according to any one of claims 2 to 8, characterized in that sodium 2-ethylhexanoate is used as the source of sodium ions.

10. Process according to any one of claims 2 to 9, characterized in that the solvent used for the crystallization of the solvate is a halogenated hydrocarbon, an ester, an alcohol or a nitrile, or a mixture of two or more of these solvents.

11. Process according to claim 10, characterized in that the solvent used for the crystallization of the solvate is dichloromethane, 1,2-dichloroethane, ethyl acetate, methyl acetate, ethanol, propanol or acetonitrile, or a mixture of two or more of these solvents.

12. Process according to claim 11, characterized in that first ethanol is added and subsequently propan-2-ol.

13. Process for the preparation of injectable preparations, characterized in that the solvate of sodium amoxicillin with N-methyl-2-pyrrolidone is made up into an injectable preparation by methods customarily employed in pharmacy.

**Claims for the Contracting State: AT**

1. Process for the preparation a crystalline solvate of sodium amoxicillin with N-methyl-2-pyrrolidone, characterized in that amoxicillin is reacted with a solution of a source of sodium ions and the solvate is subsequently caused to crystallize by addition of one or more organic solvents in the presence of N-methyl-2-pyrrolidone.

2. Process according to claim 1, characterized in that amoxicillin is used in the trihydrate form.

3. Process according to claim 1 or 2, characterized in that the reaction between amoxicillin and the source of sodium ions is carried out in a polar organic solvent.

4. Process according to claim 3, characterized in that N-methyl-2-pyrrolidone or a mixture thereof with a polar organic solvent is used as the polar organic solvent.

5. Process according to claim 4, characterized in that a mixture of N-methyl-2-pyrrolidone with an alcohol having at most 6 carbon atoms is used as the polar organic solvent.

6. Process according to any one of claims 1 to 5, characterized in that the reaction and the crystallization of the solvate are carried out at a temperature between −10 and +40° centigrade.

7. Process according to claim 6, characterized in that the reaction and the crystallization of the solvate are carried out at a temperature between 0 and 25° centigrade.

8. Process according to any one of claims 1 to 7, characterized in that sodium 2-ethylhexanoate is used as the source of sodium ions.

9. Process according to any one of claims 1 to 8, characterized in that the solvent used for the crystallization of the solvate is a halogenated hydrocarbon, an ester, an alcohol or a nitrile, or a mixture of two or more of these solvents.

10. Process according to claim 9, characterized in that the solvent used for the crystallization of the solvate is dichloromethane, 1,2-dichloroethane, ethyl acetate, methyl acetate, ethanol, propanol or acetonitrile, or a mixture of two or more of these solvents.

11. Process according to claim 10, characterized in that first ethanol is added and subsequently propan-2-ol.

12. Process for the preparation of injectable preparations, characterized in that the solvate of sodium amoxicillin with N-methyl-2-pyrrolidone is made up into an injectable preparation by methods customarily employed in pharmacy.

**Revendications pour les etats contractants: BE, CH-LI, DE, FR, SB, JT, LU, NL, SE**

1. Solvate cristallin de l'amoxicilline sodique avec la N-méthyl-2-pyrrolidone.

2. Procédé de préparation du solvate suivant la revendication 1, caractérisé en ce qu'on fait réagir l'amoxicilline avec une solution d'une source d'ions sodium et on fait cristalliser le solvate ensuite par addition d'un ou plusieurs solvants organiques en présence de N-méthyl-2-pyrrolidone.

3. Procédé suivant la revendication 2, caractérisé en ce qu'on utilise l'amoxicilline sous la forme du trihydrate.

4. Procédé suivant la revendication 2 ou 3, caractérisé en ce qu'on exécute la réaction entre l'amoxicilline et la source d'ions sodium dans un solvant organique polaire.

5. Procédé suivant la revendication 4, caractérisé en ce qu'on utilise la N-méthyl-2-pyrrolidone ou un mélange de celle-ci avec un solvant organique polaire comme solvant organique polaire.

6. Procédé suivant la revendication 5, caractérisé en ce qu'on utilise um mélange de N-méthyl-2-pyrrolidone avec un alcool comptant au maximum 6 atomes de carbone comme solvant organique polaire.

7. Procédé suivant l'une quelconque des revendications 2 à 6, caractérisé en ce qu'on exécute la réaction et la cristallisation du solvate à une température située entre −10 et +40°C.

8. Procédé suivant la revendication 7, caractérisé en ce qu'on exécute la réaction et la cristallisation du solvate à une température située entre 0 et 25°C.

9. Procédé suivant l'une quelconque des revendications 2 à 8, caractérisé en ce qu'on utilise le 2-éthylhexanoate de sodium comme source d'ions sodium.

10. Procédé suivant l'une quelconque des revendications 2 à 9, caractérisé en ce que le solvant utilisé pour la cristallisation du solvate est un hydrocarbure halogéné, un ester, un alcool ou un nitrile ou bien un mélange de deux ou plusieurs de ces solvants.

11. Procédé suivant la revendication 10, caractérisé en ce que le solvant utilisé pour la cristallisation du solvate est le dichlorométhane, le 1,2-dichloroéthane, l'acétate d'éthyle, l'acétate de méthyle, l'éthanol, le propanol ou l'acétonitrile ou bien un mélange de deux ou plusieurs de ces solvants.

12. Procédé suivant la revendication 11, caractérisé en ce qu'on ajoute d'abord de l'éthanol et ensuite du propane-2-ol.

13. Procédé de préparation de compositions injectables, caractérisé en ce qu'on convertit le solvate de l'amoxicilline sodique avec la N-méthyl-2-pyrrolidone en une composition injectable suivant des procédés d'application courante en pharmacie.

**Revendications pour l'etat contractant: AT**

1. Procédé de préparation d'un solvate cristallin de l'amoxicilline sodique avec la N-méthyl-2-pyrrolidone, caractérisé en ce qu'on fait réagir l'amoxicilline avec une solution d'une source d'ions sodium et on fait cristalliser le solvate ensuite par addition d'un ou plusieurs solvants organiques en présence de N-méthyl-2-pyrrolidone.

2. Procédé suivant la revendication 1, caractérisé en ce qu'on utilise l'amoxicilline sous la forme du trihydrate.

3. Procédé suivant la revendication 1 ou 2, caractérisé en en ce qu'on exécute la réaction entre l'amoxicilline et la source d'ions sodium dans un solvant organique polaire.

4. Procédé suivant la revendication 3, caractérisé en ce qu'on utilise la N-méthyl-2-pyrrolidone ou un mélange de celle-ci avec un solvant organique polaire comme solvant organique polaire.

5. Procédé suivant la revendication 4, caractérisé en ce qu'on utilise un mélange de N-méthyl-2-pyrrolidone avec un alcool comptant au maximum 6 atomes de carbone comme solvant organique polaire.

6. Procédé suivant l'une quelconque des revendications 1 à 5, caractérisé en ce qu'on exécute la réaction et la cristallisation du solvate à une température située entre —10 et +40°C.

7. Procédé suivant la revendication 6, caractérisé en ce qu'on exécute la réaction et la cristallisation du solvate à une température située entre 0 et 25°C.

8. Procédé suivant l'une quelconque des revendications 1 à 7, caractérisé en ce qu'on utilise le 2-éthylhexanoate de sodium comme source d'ions sodium.

9. Procédé suivant l'une quelconque des revendications 1 à 8, caractérisé en ce que le solvant utilisé pour la cristallisation du solvate est un hydrocarbure halogéné, un ester, un alcool ou un nitrile ou bien un mélange de deux ou plusieurs de ces solvants.

10. Procédé suivant la revendication 9, caractérisé en ce que le solvant utilisé pour la cristallisation du solvate est le dichlorométhane, le 1,2-dichloroéthane, l'acétate d'éthyle, l'acétate de méthyle, l'éthanol, le propanol ou l'acétonitrile ou bien un mélange de duex ou plusieurs de ces solvants.

11. Procédé suivant la revendication 10, caractérisé en ce qu'on ajoute d'abord de l'éthanol et ensuite du propane-2-ol.

12. Procédé de préparation de compositions injectables, caractérisé en ce qu'on convertit le solvate de l'amoxicilline sodique avec la N-méthyl-2-pyrrolidone en une composition injectable suivant des procédés d'application courante en pharmacie.

**Patentansprüche für die Vertragsstaaten: BE, CH-LI, DE, FR, SB, JT, LU, NL, SE**

1. Kristallines Solvat von Natrium-amoxicillin mit N-Methyl-2-pyrrolidon.

2. Verfahren zur Herstellung des in Anspruch 1 beanspruchten Solvats, dadurch gekennzeichnet, daß Amoxicillin mit einer Lösung einer Quelle von Natriumionen umgesetzt und das Solvat anschließend durch Zusatz von einem oder mehreren organischen Lösungsmitteln in Gegenwart von N-Methyl-2-pyrrolidon zur Kristallisation gebracht wird.

3. Verfahren nach Anspruch 2, dadurch gekennzeichnet, daß Amoxicillin in der Trihydratform verwendet wird.

4. Verfahren nach Anspruch 2 oder 3, dadurch gekennzeichnet, daß die Umsetzung zwischen Amoxicillin und der Quelle von Natriumionen in einem polaren organischen Lösungsmittel durchgeführt wird.

5. Verfahren nach Anspruch 4, dadurch gekennzeichnet, daß als polares organisches Lösungsmittel N-Methyl-2-pyrrolidon oder eine Mischung davon mit einem polaren organischen Lösungsmittel verwendet wird.

6. Verfahren nach Anspruch 5, dadurch gekennzeichnet, daß als polares organisches Lösungsmittel eine Mischung von N-Methyl-2-pyrrolidon mit einem Alkohol, der höchstens 6 Kohlenstoffatome aufweist, verwendet wird.

7. Verfahren nach einem der Ansprüche 2 bis 6, dadurch gekennzeichnet, daß die Umsetzung und die Kristallisation des Solvats bei einer Temperatur zwischen —10 und +40°C durchgeführt werden.

8. Verfahren nach Anspruch 7, dadurch gekennzeichnet, daß die Umsetzung und die Kristallisation des Solvats bei einer Temperatur zwischen 0 und 25°C durchgeführt werden.

9. Verfahren nach einem der Ansprüche 2 bis 8, dadurch gekennzeichnet, daß Natrium-2-ethylhexanoat als Quelle der Natriumionen verwendet wird.

10. Verfahren nach einem der Ansprüche 2 bis 9, dadurch gekennzeichnet, daß das für die Kristallisation des Solvats verwendete Lösungsmittel ein halogenierter Kohlenwasserstoff, ein Ester, ein Alkohol oder ein Nitril oder eine Mischung von zwei oder mehreren dieser Lösungsmittel ist.

11. Verfahren nach Anspruch 10, dadurch gekennzeichnet, daß das für die Kristallisation des Solvats verwendete Lösungsmittel Dichlormethan, 1,2-Dichlorethan, Ethylacetat, Methylacetat, Ethanol, Propanol oder Acetonitril oder eine Mischung von zwei oder mehreren dieser Lösungsmittel ist.

12. Verfahren nach Anspruch 11, dadurch gekennzeichnet, daß zuerst Ethanol und anschließend Propan-2-ol zugesetzt wird.

13. Verfahren zur Herstellung von injizierbaren Zubereitungen, dadurch gekennzeichnet, daß das Solvat von Natrium-amoxicillin mit N-Methyl-2-pyrrolidon mit Hilfe von in der Pharmazie üblicherweise angewandten Methoden in eine injizierbare Zubereitung übergeführt wird.

**Patentansprüche für den Vertragsstaat Österreich**

1. Verfahren zur Herstellung eines kristallinen Solvats von Natrium-amoxicillin mit N-Methyl-2-pyrrolidon, dadurch gekennzeichnet, daß Amoxicillin mit einer Lösung einer Quelle von Natriumionen umgesetzt und das Solvat anschließend durch Zusatz von einem oder mehreren organischen Lösungsmitteln in Gegenwart von N-Methyl-2-pyrrolidon zur Kristallisation gebracht wird.

2. Verfahren nach Anspruch 1, dadurch gekennzeichnet, daß Amoxicillin in der Trihydratform verwendet wird.

3. Verfahren nach Anspruch 1 oder 2, dadurch gekennzeichnet, daß die Umsetzung zwischen Amoxicillin und der Quelle von Natriumionen in einem polaren organischen Lösungsmittel durchgeführt wird.

4. Verfahren nach Anspruch 3, dadurch gekennzeichnet, daß N-Methyl-2-pyrrolidon oder eine Mischung davon mit einem polaren organischen Lösungsmittel als polares organisches Lösungsmittel verwendet wird.

5. Verfahren nach Anspruch 4, dadurch gekennzeichnet, daß eine Mischung von N-Methyl-2-pyrrolidon mit einem Alkohol, der höchstens 6 Kohlenstoffatome aufweist, als polares organisches Lösungsmittel verwendet wird.

6. Verfahren nach einem der Ansprüche 1 bis 5, dadurch gekennzeichnet, daß die Umsetzung und die Kristallisation des Solvats bei einer Temperatur zwischen −10 und +40°C durchgeführt werden.

7. Verfahren nach Anspruch 6, dadurch gekennzeichnet, daß die Umsetzung und die Kristallisation des Solvats bei einer Temperatur zwischen 0 und 25°C durchgeführt werden.

8. Verfahren nach einem der Ansprüche 1 bis 7, dadurch gekennzeichnet, daß Natrium-2-ethylhexanoat als Quelle der Natriumionen verwendet wird.

9. Verfahren nach einem der Ansprüche 1 bis 8, dadurch gekennzeichnet, daß das für die Kristallisation des Solvats verwendete Lösungsmittel ein Halogenierter Kohlenwasserstoff, ein Ester, ein Alkohol oder ein Nitril oder eine Mischung von zwei oder mehreren dieser Lösungsmittel ist.

10. Verfahren nach Anspruch 9, dadurch gekennzeichnet, daß das für die Kristallisation des Solvats verwendete Lösungsmittel Dichlormethan, 1,2-Dichlorethan, Ethylacetat, Methylacetat, Ethanol, Propanol oder Acetonitril oder eine Mischung von zwei oder mehreren dieser Lösungsmittel ist.

11. Verfahren nach Anspruch 10, dadurch gekennzeichnet, daß zuerst Ethanol und anschließend Propan-2-ol zugesetzt wird.

12. Verfahren zur Herstellung von injizierbaren Zubereitungen, dadurch gekennzeichnet, daß das Solvat von Natrium-amoxicillin mit N-Methyl-2-pyrrolidon mit Hilfe von in der Pharmazie üblicherweise angewandten Methoden in eine injizierbare Zubereitung übergeführt wird.